# EUROPEAN PATENT APPLICATION

(11) **EP 4 516 221 A1**
(43) Date of publication of application: **05.03.2025**
(21) Application number: 23214345.3
(22) Date of filing: 05.12.2023
(51) Int. Cl.: A61B 5/1455, A61B 5/055

(54) **PULSE OXIMETRY IN MAGENTIC RESONANCE ENVIRONMENTS UTILIZING HYBRID FIBER OPTIC CABLES**

(30) Priority: 29.08.2023 US 202363535085 P
(71) Applicant: Koninklijke Philips N.V., 5656 AG Eindhoven (NL)
(72) Inventor: KOSTAKIS, Dimitri George, Eindhoven (NL); MARTIN, Christian, Eindhoven (NL); BRADY, Leigh, 5656AG Eindhoven (NL)
(74) Representative: Philips Intellectual Property & Standards

(57) **Abstract**

While pulse oximetry is a frequently used patient monitoring technique to measure blood oxygen saturation, convention pulse oximetry devices often contain materials and/or mechanisms that are negatively affected by high magnetic fields, thereby preventing reliable results and/or creating additional health risks to patients. Thus, the present disclosure describes a pulse oximetry device adapted for use in high-magnetic field environments, wherein the pulse oximetry device comprises a wearable element having a radiation conveying element and one or more photodetectors, and the wearable element is connected to an associated measurement device via a hybrid cable comprising a fiberoptic bundle and high impedance cabling. Methods of performing pulse oximetry using the devices and hybrid cable are also described.

## Description

### FIELD OF THE INVENTION

The present disclosure relates generally to pulse oximetry devices and methods of performing pulse oximetry, and more specifically to pulse oximetry devices adapted for high-magnetic field environments and methods of performing pulse oximetry in such environments.

### BACKGROUND OF THE INVENTION

Pulse oximetry is a frequently-used patient measurement technique that measures blood oxygen saturation (SpO2), and generally involves sending light through the tissues of a patient, such as through the patient's finger, toe, ear, nose, and/or other extremity, and measuring the resulting light passing through those tissues. The result is an oxygen level typically displayed as a percentage on a patient monitoring system.

Conventionally, conductive metal wiring is used to power one or more light sources located on the device attached to the patient, and a photodiode also located on the device is used to receive the resulting light emissions. However, given the nature of magnetic resonance (MR) environments (i.e., environments subject to high magnetic fields due to, for example, medical devices like a magnetic resonance imaging machine), conductive wiring creates significant technical challenges as well as risks to the patient. For example, conductive wiring is rarely used in such environments due to the risk of looping, undesirable antenna effects, and resulting radio-frequency (RF) heating effect of the cabling. For these and other reasons, conventional pulse oximetry devices may not be suitable for use in MR environments.

### SUMMARY OF THE INVENTION

While pulse oximetry is a frequently used patient monitoring technique to measure blood oxygen saturation, conventional pulse oximetry devices often contain materials and/or mechanisms that are negatively affected by high magnetic fields, thereby preventing reliable results and/or creating additional health risks to patients. Nevertheless, it remains desirable, in certain situations, to measure blood oxygen saturation while the patient and medical device are subject to a high magnetic field. Accordingly, provided herein are pulse oximetry devices and methods of performing pulse oximetry that utilizes a combination cable having fiberoptic cabling and high-impedance cabling that minimizes the cable assembly while still being compatible with high magnetic field environments.

According to one embodiment of the present disclosure, a pulse oximetry device adapted for use in high-magnetic field environments is provided. The device can include: a wearable element having a radiation conveying element and one or more photodetectors, wherein the radiation conveying element and the one or more photodetectors are arranged relative to the wearable element such that the radiation conveying element transmits one or more wavelengths of radiation to a tissue of a subject and is received by the one or more photodetectors when the wearable element is worn by the subject; and a hybrid cable configured to connect to the wearable element, wherein the hybrid cable includes a fiberoptic bundle configured to transmit one or more wavelengths of radiation from one or more radiation sources to the radiation conveying element when connected to the wearable element, and a high-impedance cabling configured to transmit electrical signals from at least one of the one or more photodetectors to an associated measurement device when connected to the wearable element.

In an aspect, the one or more radiation sources may include a first radiation source configured to generate radiation having a wavelength between about 620 nanometers and about 750 nanometers.

In an aspect, the one or more radiation sources may include a second radiation source configured to generate radiation having a wavelength between about 780 nanometers and about 1 millimeter.

In an aspect, the hybrid cable may be a removable cable configured to reversibly connect to the wearable element.

In an aspect, the hybrid cable may include a connector configured to connect the fiberoptic bundle with the radiation conveying element, and configured to connect the high-impedance cabling with the one or more photodetectors.

In an aspect, the wearable element may further include a locking element configured to secure the connector of the hybrid cable to the wearable element.

In an aspect, the hybrid cable may include an outer jacket encasing the fiberoptic bundle and the high-impedance cabling.

In an aspect, the high-impedance cabling may include one or more protected, electrically-conductive wires, each protected, electrically-conductive wire being configured to transmit an electrical signal from one of the one or more photodetectors to the associated measurement device when the hybrid cable is connected to the wearable element.

In an aspect, the one or more photodetectors of the wearable element may include at least a first photodetector and a second photodetector, the high-impedance cabling may include at least a first protected, electrically-conductive wire and a second protected, electrically-conductive wire, the first protected, electrically-conductive wire is configured to transmit a first electrical signal from the first photodetector to the associated measurement device, and the second protected, electrically-conductive wire is configured to transmit a second electrical signal from the second photodetector to the associated measurement device.

According to another embodiment of the present disclosure, a hybrid cable adapted for performing pulse oximetry in high-magnetic field environments is provided. The hybrid cable can include: a fiberoptic bundle configured to transmit one or more wavelengths of radiation from one or more radiation sources to a radiation conveying element of a wearable element of a pulse oximetry device; and a high-impedance cabling configured to transmit electrical signals from one or more photodetectors of the wearable element of the pulse oximetry device to an associated measurement device.

In an aspect, the one or more radiation sources are located on the associated measurement device.

In an aspect, the high-impedance cabling may include at least a first protected, electrically-conductive wire and a second protected, electrically-conductive wire, each protected, electrically-conductive wire being configured to transmit electrical signals from a corresponding photodetector of the wearable element of the pulse oximetry device to the associated measurement device.

In an aspect, the hybrid cable may include an outer jacket encasing the fiberoptic bundle and the high-impedance cabling.

In an aspect, the hybrid cable may further include a connector configured to connect the fiberoptic bundle with a radiation conveying element, and configured to connect the high-impedance cabling with the one or more photodetectors when the connector is engaged with the wearable element of the pulse oximetry device.

According to another embodiment of the present disclosure, a method of performing pulse oximetry in a high-magnetic field environment using a pulse oximetry device is provided. The pulse oximetry device can include (i) a wearable element having a radiation conveying element and one or more photodetectors, and (ii) a hybrid cable connected to the wearable element, wherein the hybrid cable includes a fiberoptic bundle and a high-impedance cabling. The method can include: transmitting one or more wavelengths of radiation to a tissue of a subject wearing the wearable element of the pulse oximetry device, wherein the one or more wavelengths of radiation are transmitted from one or more radiation sources through the fiberoptic bundle of the hybrid cable and to the tissue of the subject via the radiation conveying element of the wearable element; receiving, via the one or more photodetectors, a radiation-based signal comprising radiation transmitted through the tissue of the subject via the radiation conveying element; and transmitting one or more electrical signals from the one or more photodetectors to an associated measurement device via the high-impedance cabling of the hybrid cable.

These and other aspects of the various embodiments will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
Fig. 1 is a diagram of a patient and pulse oximetry device in an environment subject to a high magnetic field shown in accordance with aspects of the present disclosure.
Fig. 2 is a block diagram of a pulse oximetry device shown in accordance with aspects of the present disclosure.
Fig. 3 is an illustration of a pulse oximetry device shown in accordance with further aspects of the present disclosure.
Fig. 4 is an illustration of a hybrid cable shown in accordance with aspects of the present disclosure.
Fig. 5 are graphs illustrating the performance of a high impedance cable for use in the pulse oximetry devices of the present disclosure.
Fig. 6 is another graph illustrating the performance of a high impedance cable for use in the pulse oximetry devices of the present disclosure.
Fig. 7A is an illustration of a pulse oximetry device shown in accordance with aspects of the present disclosure.
Fig. 7B is an illustration of the hybrid cable used in connection with the device shown in Fig. 7A.
Fig. 8 is a flowchart illustrating a method of measuring blood oxygen saturation in a high magnetic field environment using a pulse oximetry device in accordance with aspects of the present disclosure.

### DETAILED DESCRIPTION OF EMBODIMENTS

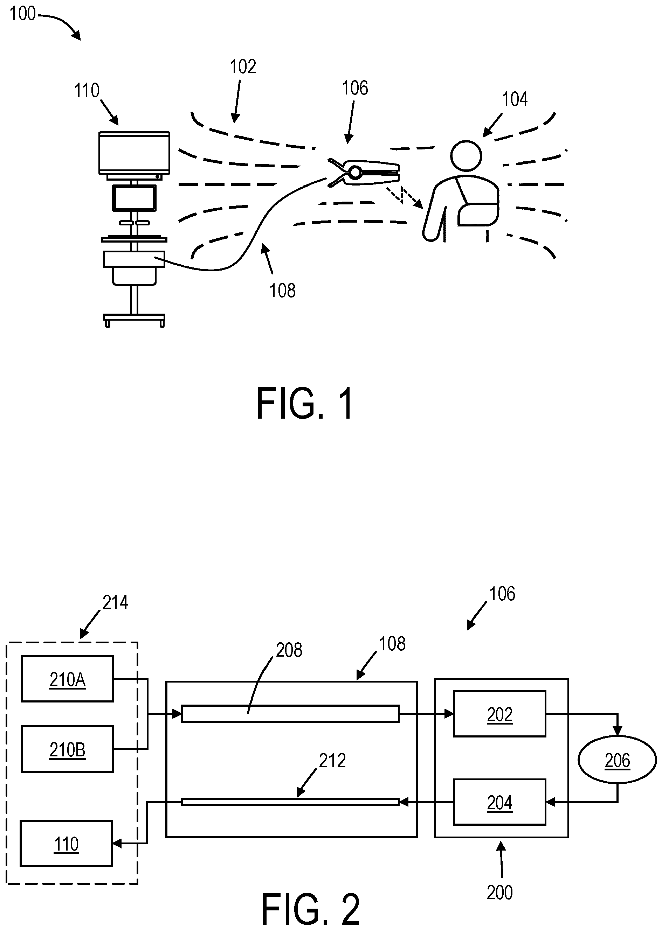

With reference to Fig. 1, an environment 100 subject to a high magnetic field 102 is illustrated according to various aspects of the present disclosure. In embodiments, the high magnetic field 102 may be generated by a magnetic field source (not shown) that generates an associated magnetic field 102. In various embodiments, the magnetic field source can be a magnetic resonance imaging (MRI) machine, however, other magnetic field sources are contemplated. The subject 104 may be located within the magnetic field source itself (such as in the case of an MRI machine), or may be located nearby to the magnetic field source but within the magnetic field 102 generated by the magnetic field source. Similarly, as shown, one or more medical devices may be located with the subject 104 within the magnetic field 102, including but not limited to a pulse oximetry device 106. As described in more detail below, a hybrid cable 108 may also be within the magnetic field 102 and connect the pulse oximetry device 106 to a measurement device 110, such as a patient monitor. In embodiments, the measurement device 110 may be adapted for use within the magnetic field 102. In other embodiments, the measurement device 110 may be outside of the range of the high magnetic field 102 generated by the magnetic field source (e.g., in another room, etc.).

According to various embodiments, the magnetic field source (not shown) may produce an associated magnetic field 102 (sometimes symbolized as *B̅*), having a direction relative to the magnetic field source and/or the environment 100 subjected to the magnetic field 102. In further embodiments, the magnetic field 102 may have a direction relative to the subject 104 and/or any medical devices within the environment 100.

Additionally, the subject 104 and/or any medical devices within the environment 100 may further experience the associated magnetic field 102 having a field strength (e.g., magnetic flux density). For example, in some embodiments, the associated magnetic field 102 may have a magnetic flux density that is greater than about 1000 gauss (about 0.1 Tesla), including greater or equal to than about 5,000 gauss (i.e., about 0.5 Tesla), greater than or equal to about 10,000 gauss (i.e., about 1 Tesla), greater than or equal to about 15,000 gauss (i.e., about 1.5 Tesla), greater than or equal to about 20,000 gauss (i.e., about 2 Tesla), and/or greater than or equal to about 30,000 gauss (i.e., about 3 Tesla).

Turning to Fig. 2, a representative illustration of a pulse oximetry device 106 and a hybrid cable 108 that are adapted for use in environments 100 subject to high magnetic fields 102 is shown in accordance with certain aspects of the present disclosure. In embodiments, the pulse oximetry device 106 comprises a wearable element 200 having a radiation conveying element 202 and one or more photodetectors 204. The wearable element 200 may be variously embodied as a clip, band, patch, wrap, and/or another device suitable for securing to an intended portion of the subject 104. For example, the wearable element 200 may be configured to secure to an extremity 206 of the subject 104, including but not limited to, a finger, toe, ear, nose, and/or the like.

In embodiments, the radiation conveying element 202 and the one or more photodetectors 204 are arranged relative to the wearable element 200 such that the radiation conveying element 202 can transmit one or more wavelengths of radiation through the extremity 206 of the subject 104 and be received by one or more of the photodetectors 204, as shown in Fig. 2. Put another way, when the wearable element 200 is worn by the subject 102 (e.g., on the finger, toe, ear, nose, etc. of the subject 102), the radiation conveying element 202 may transmit one or more wavelengths of radiation to that extremity 206 and a corresponding signal is received by one or more of the photodetectors 204.

As described herein, one or more wavelengths of radiation may be transmitted through the tissues of the extremity 206 and measured by the photodetectors 204 (i.e., transmissive mode), and/or one or more wavelengths of radiation may be reflected by the tissues of the extremity 206 and measured by the photodetectors 204 (i.e., reflective mode). That is, in particular embodiments, the each of the one or more photodetectors 204 can be configured to detect radiation such as one or more wavelengths of light that are transmitted through and/or reflected by the tissues of the extremity 206 of the patient 102. In embodiments, each photodetector 204 is configured to detect light within a specific range of wavelengths. In some embodiments, one or more of the photodetectors can be photodiodes that converts light received through the extremity 206 of the subject 104 from the radiation conveying element 202 into an electrical voltage or photocurrent. The wavelengths of light converted by the one or more photodetectors 204 can then be transmitted to the measurement device 110 via the high impedance cabling 212 (described in more detail below).

In embodiments, the pulse oximetry device 106 may further include a hybrid cable 108 configured to connect to the wearable element 200. In particular embodiments, the hybrid cable 108 comprises a fiberoptic bundle 208 configured to carry or otherwise transmit one or more wavelengths of radiation (i.e., light) from one or more radiation sources 210A, 210B to the radiation conveying element 202, as shown in Fig. 2. In further embodiments, the hybrid cable 108 comprises a high impedance cabling 212 configured to carry or otherwise transmit electrical signals from one or more photodetectors 204 to an associated measurement device 110. Put another way, fiberoptic bundle 208 of the hybrid cable 108 may be configured to operatively connect one or more radiation sources 210A, 210B with the radiation conveying element 202 of the wearable element 200, while the high impedance cabling 212 of the hybrid cable 108 may be configured to operatively connect one or more photodetectors 204 of the wearable element 200 to an associated measurement device 110.

In particular embodiments, the hybrid cable 108 may be a removable cable that is configured to reversibly connect to the wearable element 200. That is, in certain embodiments, the hybrid cable 108 may be attached and/or detached from the wearable element 200 as needed to suit clinical needs.

As described herein, the radiation sources 210A, 210B are configured to generate or otherwise produce one or more wavelengths of radiation, such as visible and/or non-visible light, which are they transmitted or otherwise carried to the wearable element 200 via the fiberoptic bundle 208 and through the extremity 206 of the subject 104 via the radiation conveying element 202. In embodiments, the one or more radiation sources 210A, 210B can include at least a first radiation source 210A configured to generate radiation having a wavelength between about 620 nanometers and about 750 nanometers, including about 660 nanometers. In further embodiments, the one or more radiation sources 210A, 210B can include at least a second radiation source 210B configured to generate radiation having a wavelength between about 780 nanometers and about 1 millimeter, including about 940 nanometers.

In embodiments, the one or more radiation sources 210A, 210B may be arranged as part of a separate device 214 that is remote from the subject 104. In particular embodiments, the separate device 214 may include the measurement device 110, as shown in Fig. 2. For example, it is contemplated that the hybrid cable 108 operatively connects the components 202, 204 of the wearable element 200 to a single device 214 that includes both the measurement device 110 and the radiation sources 210A, 210B. However, it is also contemplated that the radiation sources 210A, 210B are separate from the measurement device 110, and the hybrid cable 108 separately connected to each of these components.

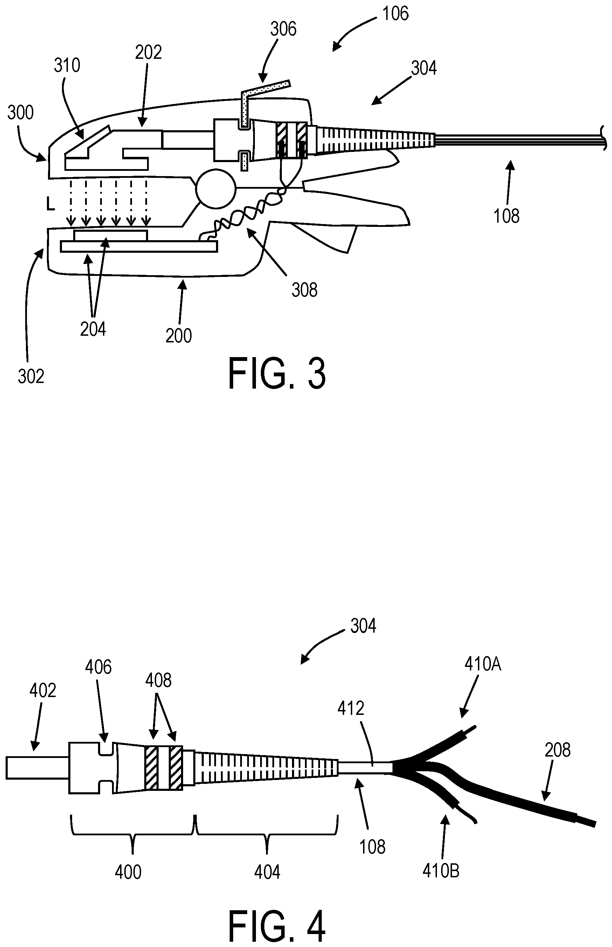

Turning to Fig. 3, a diagram is provided that illustrates an exemplary embodiment of a pulse oximetry device 106 having a hybrid cable 108 attached thereto in accordance with various aspects of the present disclosure. In particular, a cross-sectional side view of a pulse oximetry device 106 showing certain inner components of the device 106 are illustrated along with the hybrid cable 108 in an engaged configuration (i.e., engaged with the wearable portion 200). As shown, the pulse oximetry device 106 includes a wearable element 200, represented in this embodiment as an actuatable finger clip. The wearable element 200 includes a radiation conveying element 202 disposed within an upper portion 300 of the wearable element 200, and multiple photodetectors 204 disposed within a lower portion 302 that opposes the upper portion 300. Put another way, the radiation conveying element 202 and the one or more photodetectors 204 are arranged relative to the wearable element 200 such that one or more wavelengths of radiation (e.g., light L shown in Fig. 3) will pass through the finger 106 of the subject 104 and be received by the one or more photodetectors 204 when the wearable element 200 is worn by the subject 104.

In embodiments, the hybrid cable 108 may include a connector 304 that is configured to connect the components of the hybrid cable 108 with the components of the wearable element 200. More specifically, in particular embodiments, the connector 304 may be configured to operatively connect the fiberoptic bundle 208 of the hybrid cable 108 to the radiation conveying element 202 of the wearable element 200. The connector 304 may also be configured to operatively connect one or more of the photodetectors 204 to the high impedance cabling 212.

In embodiments, the connector 304 of the hybrid cable 108 may engage a locking mechanism 306 of the wearable element 200 such that the hybrid cable 108 is securely and operatively connected to the components of the wearable element 200. In particular embodiments, the locking mechanism 306 may configured to be releasable. That is, in embodiments, the hybrid cable 108 may be a removable cable configured to reversibly connect to the wearable element 200.

Although various embodiments are contemplated, an exemplary view of a connector 304 of a hybrid cable 108 is illustrated in Fig. 4. As shown, the connector 304 includes a housing 400 providing one or more features, a radiation source fiberoptic portion 402 providing a conduit to the radiation conveying element 202, and a strain relief portion 404 providing strain relief to the hybrid cable 108. In embodiments, the housing 400 may provide a locking feature 406 corresponding to the locking mechanism 306 of the wearable element 200. For example, the locking mechanism 306 can be an actuatable pin or set of pins that reversibly engage a locking feature 406 (e.g., a recess as shown in Fig. 4) of the connector 304.

In embodiments, the housing 400 may also include electrical interfaces 408 configured to communicate electrical signals to the high impedance cabling 212. For example, as shown in Figs. 3 and 4, the electrical interfaces 408 may be ring conduits that engage wiring 308 of the wearable element 200, thereby connecting one or more photodetectors 204 to the hybrid cable 108. In embodiments, each of the photodetectors 204 may be connected to a separate electrical interface 408. Thus, in embodiments, the number of electrical interfaces 408 may correspond to the number of photodetectors 204 located on the wearable element 200. However, it should be appreciated that although two electrical interfaces 408 are shown in Figs. 3 and 4, the wearable element 200 may include additional photodetectors that can also be connected to additional electrical interfaces. For example, in some embodiments, the wearable element 200 may include a source photodetector 310 that is also connected to an additional electrical interface (not shown) of the connector 304.

As described above, the hybrid cable 108 can include a fiberoptic bundle 208 and high impedance cabling 212. More specifically, as shown in Fig. 4, the high impedance cabling 212 may comprise one or more high impedance wires 410A, 410B, including at least two high impedance wires 410A, 410B. It should be appreciated that the number of high impedance wires 410A, 410B may be based on the number of photodetectors 204, such that each high impedance wire 410A, 410B carries a separate electrical signal from a corresponding photodetector 204. For example, in embodiments, the wearable element 200 may include one photodetector 204 and the high-impedance cabling 212 may include two conductive wires 410A, 410B operatively connected to the single photodetector 204. In further embodiments, the wearable element 200 may include two or more photodetectors 204 and the high-impedance cabling 212 can include three or more conductive wires 410A, 410B operatively connected to at least one of the photodetectors 204.

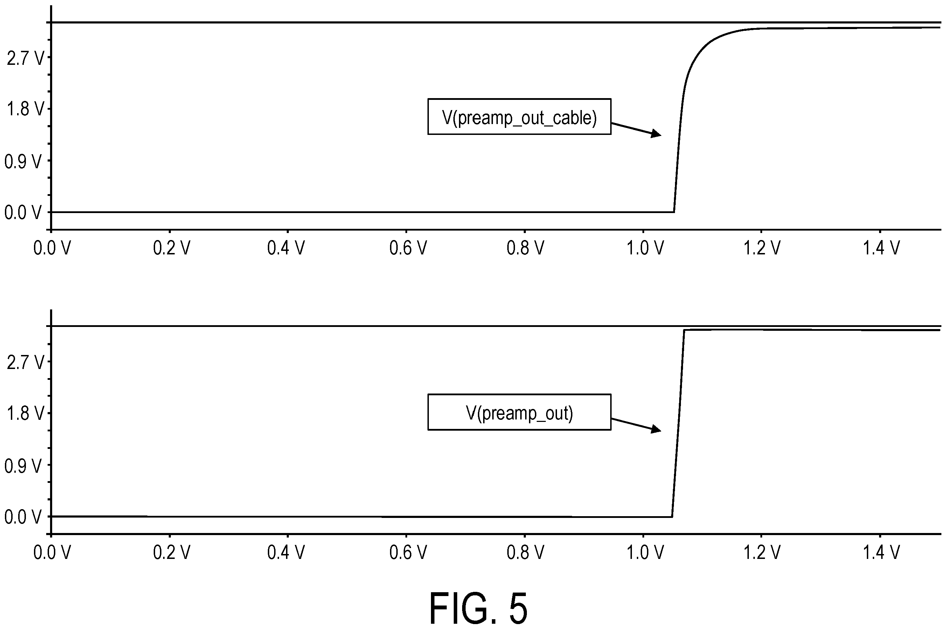

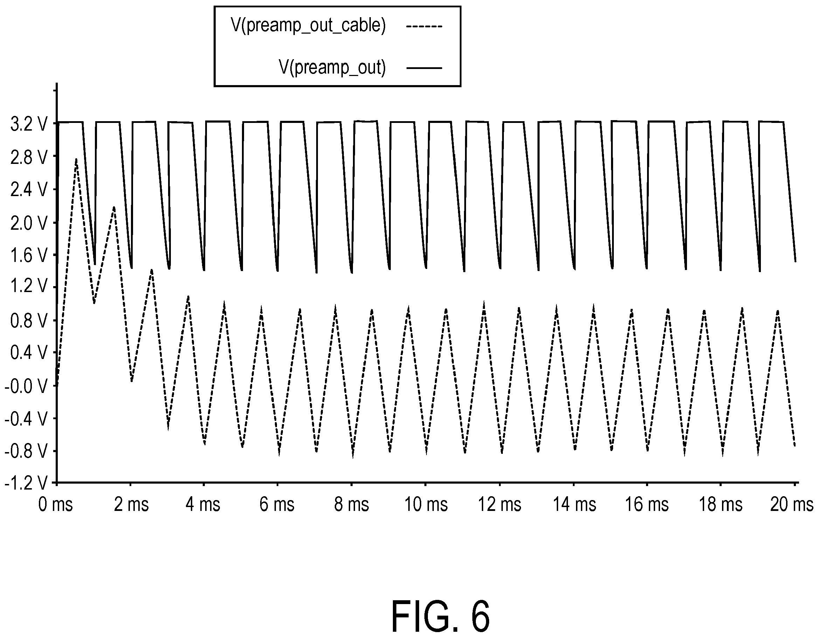

In embodiments, each of the high impedance wires 410A, 410B can be fully protected and/or shielded conductive wires. For example, in various embodiments, the high impedance wires 410A, 410B may have a resistance of at least about 5 kΩ per foot of cable, including at least about 10 kQ per foot of cable. In particular embodiments, as shown in the graphs of Figs. 5 and 6, the high impedance wires 410A, 410B can have a turn-on voltage response time that is similar to the fiberoptic bundle 208.

It should be appreciated that the wires, cables, and cabling described herein may include one or more protective and/or shielding layers, coatings, and/or jackets. For example, in particular embodiments, the fiberoptic bundle 208 may include a fiberoptic core (e.g., a continuous strand of super thin glass, etc.), a layer of cladding surrounding and reflecting light back into the core, an inner sheath surrounding the cladding (e.g., made from a high tensile strength material such as Kevlar), and an outer sheath (e.g., made from a soft plastic like polyvinylchloride). In further embodiments, the high impedance wires 410A, 410B can include an electrically-conductive wire (e.g., a metal wire comprising copper, etc.) surrounded by one or more shielding and/or insulating layers. In specific embodiments, the hybrid cable 108 comprises an outer jacket 412 encasing at least the fiberoptic bundle 208 and the high impedance cabling 212, including one or more high impedance wires 410A, 410B as shown in Fig. 4.

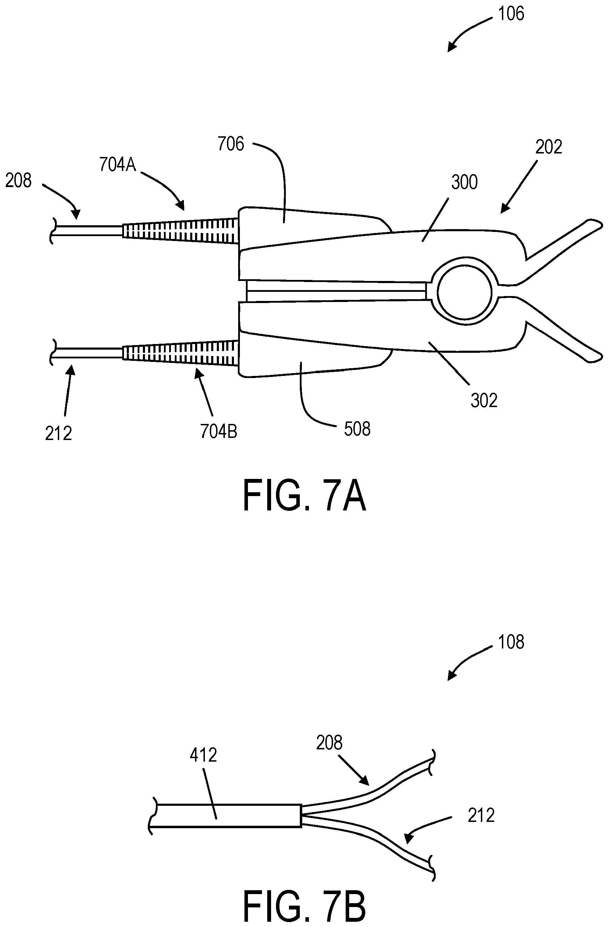

In certain embodiments, the hybrid cable 108 can include multiple connectors that engage the wearable element 200 of the pulse oximetry device 106. For example, with reference to Figs. 7A and 7B, an exemplary pulse oximetry device 106 operatively connected to a hybrid cable 108 having a first and second connector 704A, 704B is shown in accordance with certain aspects of the present disclosure. In some embodiments, a first connector 704A may operatively connect a fiberoptic bundle 208 of a hybrid cable 108 to a first port 706 of the wearable element 200. In further embodiments, a second connector 704B may operatively connect a high impedance cabling 212 of a hybrid cable 108 to a second port 708 of the wearable element 200. As shown in Fig. 7B, the fiberoptic bundle 208 and the high impedance cabling 212 may be joined together in an outer jacket 412.

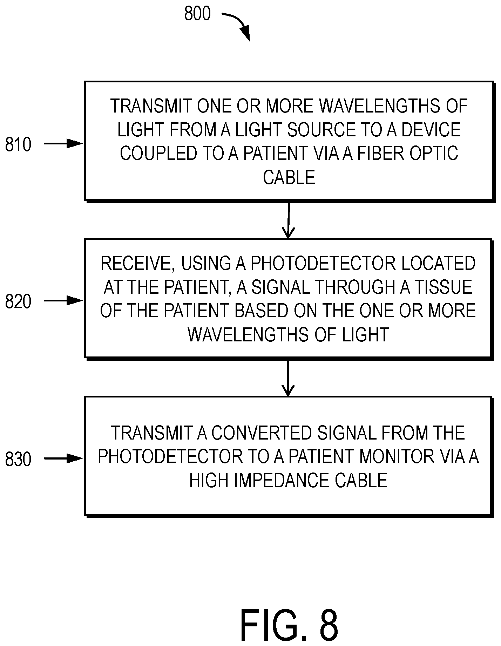

Also provided herein are methods of performing pulse oximetry in a high magnetic field environment using a pulse oximetry device 106 and hybrid cable 108 in accordance with the various aspects described in the present disclosure. For example, with reference to Fig. 8, a flowchart illustrating a method 800 of performing pulse oximetry in a high magnetic field environment is shown. The method 800 can include: in a step 810, transmitting one or more wavelengths of radiation to a tissue (i.e., extremity 206) of a subject 102 wearing a wearable element 200 of a pulse oximetry device 106; in a step 820, receiving, via one or more photodetectors 204 arranged on the wearable element 200 of the pulse oximetry device 106, an amount of radiation based on the radiation transmitted through the tissue / extremity 206 of the subject 102; and in a step 830, transmitting one or more electrical signals from the photodetectors 204 to an associated measurement device 110.

In embodiments, the step 810 includes transmitting one or more wavelengths of radiation (e.g., light) from one or more radiation / light sources 210A, 210B via a fiberoptic bundle 208, such as a fiberoptic bundle 208 of a hybrid cable 108. In certain embodiments, the radiation may be directed through the tissue / extremity 206 of the subject 102 via a radiation conveying element 202 arranged on the wearable element 200.

In further embodiments, the step 820 includes receiving an amount of radiation / light that has been transmitted through the tissue / extremity 206 of the subject 102 using one or more photodetectors 204. As described above, one or more of the detectors 204 may be photodiodes configured to convert the radiation / light received through the extremity 206 of the subject 104 into an electrical voltage or photocurrent.

In embodiments, the step 830 includes transmitting the electrical voltage or photocurrent produced by the photodetectors 204 (also referred to herein as electrical signals) to an associated measurement device 110 via high impedance cabling 208. The measurement device 110 may then convert these electrical signals into a measurement of blood oxygen saturation for the subject 104, such as a percentage indicating the blood oxygen saturation of the subject 104.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A pulse oximetry device (106) adapted for use in high-magnetic field environments (100), the device (106) comprising:
a wearable element (200) having a radiation conveying element (202) and one or more photodetectors (204), wherein the radiation conveying element (202) and the one or more photodetectors (204) are arranged relative to the wearable element (200) such that the radiation conveying element (202) transmits one or more wavelengths of radiation (L) to a tissue (206) of a subject (104) and is received by the one or more photodetectors (204) when the wearable element (200) is worn by the subject (104); and
a hybrid cable (108) configured to connect to the wearable element (200), wherein the hybrid cable (108) includes a fiberoptic bundle (208) configured to transmit one or more wavelengths of radiation from one or more radiation sources (210A, 210B) to the radiation conveying element (202) when connected to the wearable element (200), and a high-impedance cabling (212) configured to transmit electrical signals from at least one of the one or more photodetectors (204) to an associated measurement device (110) when connected to the wearable element (200).

2. The pulse oximetry device (106) of claim 1, wherein the one or more radiation sources (210A, 210B) includes a first radiation source (210A) configured to generate radiation having a wavelength between about 620 nanometers and about 750 nanometers.

3. The pulse oximetry device (106) of claim 1, wherein the one or more radiation sources (210A, 210B) includes a second radiation source (210B) configured to generate radiation having a wavelength between about 780 nanometers and about 1 millimeter.

4. The pulse oximetry device (106) of claim 1, wherein the hybrid cable (108) is a removable cable configured to reversibly connect to the wearable element (200).

5. The pulse oximetry device (106) of claim 1, wherein the hybrid cable (108) comprises a connector (304, 704A, 704B) configured to connect the fiberoptic bundle (208) with the radiation conveying element (202), and configured to connect the high-impedance cabling (212) with the one or more photodetectors (204).

6. The pulse oximetry device (106) of claim 5, wherein the wearable element (200) further comprises a locking element (306) configured to secure the connector (304, 704A, 704B) of the hybrid cable (108) to the wearable element (200).

7. The pulse oximetry device (106) of claim 1, wherein the hybrid cable (108) comprises an outer jacket (412) encasing the fiberoptic bundle (208) and the high-impedance cabling (212).

8. The pulse oximetry device (106) of claim 1, wherein the high-impedance cabling (212) comprises one or more protected, electrically-conductive wires (410A, 410B), each protected, electrically-conductive wire (410A, 410B) being configured to transmit an electrical signal from one of the one or more photodetectors (204) to the associated measurement device (110) when the hybrid cable (108) is connected to the wearable element (200).

9. The pulse oximetry device (106) of claim 8, wherein the one or more photodetectors (204) of the wearable element (200) includes at least a first photodetector and a second photodetector, the high-impedance cabling (212) comprises at least a first protected, electrically-conductive wire (410A) and a second protected, electrically-conductive wire (410B), and wherein the first protected, electrically-conductive wire (410A) is configured to transmit a first electrical signal from the first photodetector to the associated measurement device (110), while the second protected, electrically-conductive wire (410B) is configured to transmit a second electrical signal from the second photodetector to the associated measurement device (110).

10. A hybrid cable (108) adapted for performing pulse oximetry in high-magnetic field environments (100), the hybrid cable (108) comprising:
a fiberoptic bundle (208) configured to transmit one or more wavelengths of radiation from one or more radiation sources (210A, 210B) to a radiation conveying element (202) of a wearable element (200) of a pulse oximetry device (106); and
a high-impedance cabling (212) configured to transmit electrical signals from one or more photodetectors (204) of the wearable element (200) of the pulse oximetry device (106) to an associated measurement device (110).

11. The hybrid cable (108) of claim 10, wherein the one or more radiation sources (210A, 210B) are located on the associated measurement device (110).

12. The hybrid cable (108) of claim 10, wherein the high-impedance cabling (212) comprises at least a first protected, electrically-conductive wire (410A) and a second protected, electrically-conductive wire (410B), each protected, electrically-conductive wire (410A, 410B) being configured to transmit electrical signals from a corresponding photodetector (204) of the wearable element (200) of the pulse oximetry device (106) to the associated measurement device (110).

13. The hybrid cable (108) of claim 10, wherein the hybrid cable (108) comprises an outer jacket (412) encasing the fiberoptic bundle (208) and the high-impedance cabling (212).

14. The hybrid cable (108) of claim 10, further comprising a connector (304, 704A, 704B) configured to connect the fiberoptic bundle (208) with a radiation conveying element (202), and configured to connect the high-impedance cabling (212) with the one or more photodetectors (204) when the connector (304, 704A, 704B) is engaged with the wearable element (200) of the pulse oximetry device (106).

15. A method (800) of performing pulse oximetry in a high-magnetic field environment using a pulse oximetry device comprising (i) a wearable element having a radiation conveying element and one or more photodetectors, and (ii) a hybrid cable connected to the wearable element, wherein the hybrid cable includes a fiberoptic bundle and a high-impedance cabling, the method comprising:
transmitting (810) one or more wavelengths of radiation to a tissue of a subject wearing the wearable element of the pulse oximetry device, wherein the one or more wavelengths of radiation are transmitted from one or more radiation sources through the fiberoptic bundle of the hybrid cable and to the tissue of the subject via the radiation conveying element of the wearable element;
receiving (820), via the one or more photodetectors, a radiation-based signal comprising radiation transmitted through the tissue of the subject via the radiation conveying element; and
transmitting (830) one or more electrical signals from the one or more photodetectors to an associated measurement device via the high-impedance cabling of the hybrid cable.
